Europäisches Patentamt

European Patent Office

Office européen des brevets

(19)

(11) Numéro de publication : **0 424 282 B1**

(12) **FASCICULE DE BREVET EUROPEEN**

(45) Date de publication du fascicule du brevet :
03.03.93 Bulletin 93/09

(51) Int. Cl.⁵ : **A61K 7/00, A61K 7/42**

(21) Numéro de dépôt : **90420435.1**

(22) Date de dépôt : **09.10.90**

(54) **Préparation cosmétique de bronzage.**

(30) Priorité : **19.10.89 FR 8913950**

(43) Date de publication de la demande :
**24.04.91 Bulletin 91/17**

(45) Mention de la délivrance du brevet :
**03.03.93 Bulletin 93/09**

(84) Etats contractants désignés :
**BE DE GB**

(56) Documents cités :
**WO-A-87/06499**
**FR-A- 2 612 776**
**FR-A- 2 624 374**

(73) Titulaire : **ERPHAR**
**75 rue Madame**
**F-75006 Paris (FR)**

(72) Inventeur : **Cotte, Jean**
**13 Chemin du Moulin d'Arche**
**F-69450 Saint Cyr Au Mont d'Or (FR)**
Inventeur : **Pouget, Joseph**
**42 avenue de la Bourdonnais**
**F-75007 Paris (FR)**
Inventeur : **Darasse, Bruno**
**8 rue Vernier**
**F-75017 Paris (FR)**

(74) Mandataire : **Laurent, Michel et al**
**Cabinet LAURENT et CHARRAS, 20, rue Louis**
**Chirpaz B.P. 32**
**F-69131 Ecully Cedex (FR)**

## Description

L'invention concerne une nouvelle préparation cosmétique de bronzage et de protection contre le soleil.

Comme on le sait, pour cette application, on utilise généralement trois types de produits différents, respectivement : bronzant, protecteur et réparateur.

Comme on le sait, le bronzage artificiel résulte en particulier d'une réaction entre un dérivé cétonique appliqué sur la peau et les acides aminés de la peau. Il s'ensuit qu'en pratique, le bronzage réalisé par de telles compositions est généralement hétérogène, puisqu'il varie d'un sujet à l'autre et en fonction des territoires de la peau. Le hale artificiel ainsi obtenu est irrégulier et disparait rapidement dans le temps, notamment au bout de trois à quatre jours. Le bronzage naturel du à la formation de mélanine peut être renforcé par apport de tyrosine qui est l'acide aminé mélaninoformateur, mais qu'il est difficile d'associer à agent bronzant artificiel, du fait de leur interéaction au sein même de la préparation.

Pour protéger la peau pendant l'action de bronzage, on fait généralement appel à des produits à base de substances agissant comme filtres U.V. Pour obtenir l'action réparatrice, on fait appel à des compositions spécifiques évitant la déshydratation, la perte d'élasticité et la formation de radicaux libres, notammant sous l'action des rayonnements actiniques.

De la sorte, ces trois fonctions respectivement bronzante, protectrice et réparatrice, sont assurées par des préparations spécifiques. Il s'ensuit un surcoût et une complexité.

L'invention pallie ces inconvénients. Elle vise une préparation cosmétique unique, permettant simultanément le bronzage instantané-retard, une action protectrice de la peau et s'il y a lieu réparatrice.

Cette préparation cosmétique de bronzage à base de liposomes et dont la phase de dispersion contient des composés filtres et de la dihydroxyacétone (DHA), **se caractérisé** en ce que la zone interne hydrophile des liposomes contient des principes hydratants et au moins un composé dérivé de la tyrosine dont la fonction $-NH_2$ est bloquée par un radical chimique.

En d'autres termes, l'invention consiste à réaliser une préparation cosmétique de bronzage en faisant appel à des liposomes, bien connus dans le domaine de la cosmétologie, dont la zone interne hydrophile contient, en plus des principes hydratants, au moins un composé dérivé de la tyrosine dont la fonction amine primaire est bloquée par un radical chimique, afin d'éviter la réaction de la DHA dans la préparation. Cette fonction doit être libérée au niveau cutané. En d'autres termes, l'invention réside simultanément dans une sélection de liposomes particuliers et dans une sélection de composants, qui dans l'application de bronzage, permettent de remplir dans les meilleures conditions les trois fonctions, respectivement bronzante, protectrice et réparatrice, lors des expositions au soleil. Cette association judicieuse de produits, permet d'améliorer le hale artificiel, de le relayer par l'accélération du bronzage naturel et de réaliser une auto-protection de la peau par la seule interéaction des composants du produit et des constituants naturels cutanés, ce que l'on ne savait pas obtenir jusqu'alors.

Dans la description et dans les revendications :

. par "liposomes", on désigne des composés bien connus se présentant généralement sous forme de nanocapsule constituée d'une couche externe lipophile formant membrane et d'une zone interne hydrophile; ces composés liposomes ont largement été décrits dans la littérature (voir par exemple documents FR-A-2 221 122, 2 315 991 et FR-A-2 624 374) ;

. par "dihydroxyacétone (DHA), on désigne un composé chimique de formule $CH_2OH\text{-}CO\text{-}CH_2OH$, qui provoque un hale artificiel résultant d'une réaction avec les acides aminés des couches cutanées superficielles;

. par "composés filtres", on désigne des substances telles que des dérivés de l'acide cinnamique ou benzylidene-camphre ou de la benzophenone, connues pour leurs propriétés spectrophotométriques qui les font absorber dans l'ultraviolet, et permettent ainsi d'arrêter totalement ou sélectivement ce type de rayonnement ;

. par "principes hydratants", on désigne des substances hygroscopiques freinant la déshydratation cutanée, tels que les polyols, l'urée, l'acide lactique, l'acide pyrrolidone carboxylique ou le collagène ;

. par "composé dérivé de la tyrosine", on désigne tout produit dont la fonction $NH_2$ de cet aminoacide est bloquée par amidification : acétyl, propionyl ou même lipoyl (voir par exemple FR-A-2 624 374);

. par "DPHP", on désigne le dérivé dipalmitoylhydroxyproline, dont les caractéristiques physicochimiques lui permettent d'être incorporé dans la structure du liposome et dont l'efficacité cutanée se traduit par une amélioration de l'élasticité de la peau.

Avantageusement, en pratique :

- le dérivé de la tyrosine est l'acétyltyrosine (ACT), voire le propionyltyrosine (PCT) ;

- le dérivé de la tyrosine représente de 0,1 à 1% du poids de la préparation, de préférence est voisin de 0,5 % ; en effet, on a observé que si cette proportion est inférieure à 0,1 %, l'activité obtenue est négligeable ; en revanche, si cette proportion excède 0,5 %, l'amélioration obtenue plafonne, de sorte que le surcoût n'engendre aucun avantage ; comme déjà dit, si le dérivé de la tyrosine est l'acétyltyrosine (ACT), cette proportion est avantageusement voisine de 0,5 % ;

- les principes hydratants contenus dans la zone interne hydrophile des liposomes et associés aux dérivés de la tyrosine, peuvent être de tout type connu pour l'hydratation cutanée ; de préférence, on utilise des composés choisis dans le groupe comprenant les substances reconnues comme ayant une action sur l'hydratation cutanée, telles que les polyols (sorbitol), l'acide pyrolidone carboxylique, l'urée, le collagène ;

- comme déjà dit, la phase de dispersion de la préparation cosmétique (lait, crème, gel, émulsion..) contient des composés filtres et de la di hydroxy acétone (DHA) ; comme composé filtre, on peut utiliser toute substance active destinée à ne laisser passer que les rayons non irritants pour la peau ; en pratique, ces composés arrêtent essentiellement les rayonnements ultraviolets B ; avantageusement, on utilise des composés choisis dans le groupe comprenant les dérivés de l'acide cinamique, du benzylidène-camphre ou de la benzophénone ; en pratique ces composés représentent de 2 à 6 % du poids de la préparation ;

- la dihydroxyacétone (DHA) contenue dans cette phase de dispersion représente de 3 à 5 % du poids de la préparation ; on a observé que si la proportion de DHA est inférieure à 3 %, l'activité bronzante est insuffisante ;en revanche, si cette proportion dépasse les 5 % malgré ce coût, on n'observe aucune amélioration notable;

- les liposomes représentent de trois (3) à dix (10) pourcent en poids de la préparation ;

- dans une version préférée, la membrane externe lipophile des liposomes contient de la dipalmitoyl hydroxyproline (DPHP) ; cette substances molle est introduite en quantité susceptible d'être fixée sur la membrane externe lipophile des liposomes ; cette quantité varie donc en fonction de la nature des liposomes de base.

Les composants essentiels de la préparation conforme à l'invention : liposomes, ACT, DHA, DPHP, agents filtres et principes hydratants doivent être en équilibre, notamment dans la membrane.

Comme on le sait, la DHA est connue pour provoquer une action auto-bronzante artificielle par réaction avec les amino-acides contenus dans la peau ; cette coloration est comme déjà dit capricieuse et irrégulière.

La présence d'ACT dans la couche interne (cavité) des liposomes permet d'homogénéiser cette coloration par apport d'amino-acides. En effet, dans l'ACT, la fonction $-NH_2$ est bloquée. Cela permet donc d'introduire ce composé dans la dispersion (crème, lait, gel) sans que la réaction puisse se produire avec la DHA. Toutefois, sous l'effet de l'action enzymatique des enzymes cutanées, on libère ces fonctions $-NH_2$. Il s'ensuit que ces fonctions sont alors apportées in situ, permettant ainsi d'homogénéiser et d'améliorer le bronzage du à la DHA. Par ailleurs, on sait que la mélanine est le pigment de bronzage naturel. Il s'ensuit que sous l'effet de l'apport de tyrosine dont la fonction $NH_2$ a été libérée, il y a augmentation du bronzage naturel du à la mélanine, prolongeant ainsi le bronzage artificiel.

Ainsi, grâce à la sélection du composé dérivé de la tyrosine (ACT), on améliore le bronzage artificiel immédiat en libérant des fonctions amines qui réagissent avec la DHA, et on augmente également la quantité de mélanine potentielle, ce qui accélère l'action de bronzage naturel. En d'autres termes, on obtient donc grâce à cette sélection, une action instantanée et également une action retard de bronzage. Ce résultat est nouveau et avantageux.

En outre, le dérivé $-N=C\langle$ (base de Schiff) provenant de la réaction de Maillard entre la DHA et la tyrosine, engendre une action anti-oxydante et anti-radicalaire. On crée donc ainsi in situ sur la peau une protection contre les radicaux libres, qui entraîne une auto-protection naturelle de la peau, et par voie de conséquence un effet protecteur.

La DPHP était connue pour son action favorable sur l'élasticité cutanée de la peau. Son incorporation dans la membrane lipophile des liposomes permet une libération de ce composé étalé dans le temps. Cela assure une réparation optimale, complétée par la libération des principes hydratants contenus dans la phase interne des liposomes.

La manière dont l'invention peut être réalisée et les avantages qui en découlent ressortiront mieux des exemples de réalisation qui suivent.

## Exemple 1 :

De manière connue, on prépare une émulsion grasse, telle qu'une crème ou un gel, dans laquelle on incorpore à une température inférieure à 40°C, une suspension de liposomes à raison d'une concentration comprise entre trois et dix pourcent (3 et 10 %), généralement voisine de 5 %.

Les liposomes sont préparés par les techniques habituelles, notamment par un procédé classique comportant les trois phases principales suivantes :

- dissolution d'un ou plusieurs phospholipides et du DPHP choisis pour la paroi des liposomes dans un solvant volatil, par exemple le chloroforme ;
- évaporation du solvant sous pression réduite ;
- addition sous agitation d'une solution aqueuse contenant entre autres l'ACT et du sorbitol ou tout autre principe hydratant.

La quantité des différents constituants spécifiques est déterminée de façon que la concentration dans la préparation finale (liposomes incorporés dans la crème) soit en poids de :
- 0,5 % pour l'ACT ;
- 5 % pour le sorbitol ;
- 2 à 3% pour le DPHP.

Tout ou partie de l'ACT et du sorbitol se trouve dans la cavité aqueuse des liposomes. Tout ou partie du DPHP se trouve inséré dans la paroi lipophile.

La suspension de liposomes ainsi obtenue est incorporée dans la forme cosmétique adéquate : crème, lait, gel.., par mélange sous agitation à une température inférieure à 40°C.

Cette préparation contient, outre les constituants classiques permettant sa réalisation :
- 3 % en poids de DHA ;
- 2 à 5 % de substances filtres.

On obtient de la sorte une crème facile à étaler qui favorise le bronzage immédiat et retard, notamment de manière homogène, protège la peau et répare éventuellement celle-ci.

**Exemple 2** :

Dans les exemples qui suivent, les concentrations en poids des composants spécifiques du liposome, DPHP, acétyltyrosine, principe hydratant, dans la formule finale sont :
- pour la DPHP : 2 à 3 % ;
- pour l'ACT : 0,5 % ;
- pour le sorbitol : 5 %.

Du fait de la quantité de liposomes incorporée, une partie de ces constituants se retrouve non "encapsulée" dans la phase émulsionnée ou dans le gel.

De manière connue, on prépare une composition ayant les caractéristiques suivantes, les proportions étant données en poids du poids total de la préparation :

```
- suspension de liposomes (comprenant DPHP, ACT
  et principes hydratants) :                              6
  dont :
      . DPHP   QS pour          2
      . ACT    QS pour          0,5
      . sorbitol  QS pour       5
```

|  |  |
|---|---|
| - palmito stéarate polyoxyéthyléné additionné d'un alcool gras poyloxyéthyléné | 8 |
| - alcool cétylique | 1 |
| - huile de vaseline | 6 |
| - alcools de lanoline | 2 |
| - glycérine | 3,2 |
| - agents conservateurs | 0,2 |
| - DHA | 4 |
| - filtres U-V | 3 |
| - eau   QSP | 100. |

On obtient de la sorte une crème classique : émulsion huile dans eau, facile à étaler, permettant une bonne action triple:bronzante, filtrante et réparatrice.

**Exemple 3** :

Comme précédemment, on réalise une composition ayant les caractéristiques suivantes :

|  |  |  |
|---|---|---|
| - suspensions de liposomes (comprenant DPHP, ACT et principes hydratants) | | 8 |
| dont : | | |
| . DPHP QS pour | 2 | |
| . ACT   QS pour | 0,5 | |
| . sel de sodium de l'acide pyrrolidone carboxylique | 2 | |
| - palmitostéarate de glycérine et de polyoxyéthylène additionné d'un alcool gras de polyoxyéthyléné | | 8 |
| - acide stéarique | | 2 |
| - glycérides oléiques polyoxyéthylénés | | 5 |
| - palmitate d'isopropyle | | 5 |

```
- glycérine                                    3,2
- agents conservateurs                         0,2
- DHA                                          4
- filtres U-V                                  4
- eau   QSP                                    100
```

On obtient les mêmes résultats qu'à l'exemple 2.

**Exemple 4** :

On reproduit une crème gel ayant les caractéristique suivantes :

```
- suspensions de liposomes (comprenant DPHP, ACT
  et principes hydratants                      8
  dont :
       . DPHP  QS pour       2
       . ACT   QS pour       0,5
       . urée QS pour        2
- palmitostéarate de
  polyoxyéthylène glycol                       10
- huile de vaseline                            10
- lanoline                                     10
- acide stéarique                              1
- glycérol                                     16,6
- gel polyacrylique
  neutralisé                                   26
- agents conservateurs                         0,1
- DHA                                          4
- filtres U-V                                  3
- eau   QSP                                    100.
```

On obtient de la sorte une crème-gel de structure légère ayant les mêmes propriétés bronzantes, filtrantes et réparatrices qu'aux exemples 2 et 3.

La préparation cosmétique de bronzage conforme à l'invention présente de nombreux avantages par rapport à celles connues et commercialisées à ce jour. On peut citer :

- la simplicité de fabrication ;
- le fait qu'il s'agit d'une préparation unique permettant à la fois de bronzer, de protéger et de réparer, ce que l'on ne savait obtenir jusqu'alors ; ces différentes activités s'exercent dans les meilleures conditions, non seulement grâce à la complémentarité d'action due à l'association judicieuse des composés choisis, mais aussi du fait de l'interéaction de certains composants avec les constituants de la peau ;
- la facilité et la sécurité de mode d'emploi.

**Revendications**

1.  Préparation cosmétique de bronzage à base de liposomes, et dont la phase de dispersion contient des composés filtres et de la dihydroxyacétone (DHA), **caractérisée** en ce que la zone interne hydrophile des liposomes contient des principes hydratants et au moins un composé dérivé de la tyrosine, dont la fonction -NH$_2$ est bloquée par un radical chimique.

2. Préparation cosmétique de bronzage selon la revendication 1, caractérisée en ce que le dérivé de la tyrosine est choisi dans le groupe comprenant l'acétyltyrosine, le propionyltyrosine, et le lipoyltyrosine.

3. Préparation cosmétique de bronzage selon la revendication 2, caractérisée en ce que le dérivé de la tyrosine représente de 0,1 à 1 % en poids du poids de la préparation, de préférence 0,5 %.

4. Préparation cosmétique de bronzage selon l'une des revendications 1 à 3, caractérisée en ce que les principes hydratants sont choisis dans le groupe comprenant les substances hygroscopiques freinant la déshydratation cutanée choisies dans le groupe constitué par les polyols, l'urée, l'acide lactique, l'acide pyrrolidone carboxylique et le collagène.

5. Préparation cosmétique de bronzage selon l'une des revendications 1 à 4, caractérisée en ce que la parci externe lipophile des liposomes contient de la dipalmitoyl hydroxyproline (DPHP).

6. Préparation cosmétique de bronzage selon l'une des revendications 1 à 5, caractérisée en ce que la dihydroxy acétone (DHA) est introduite à raison de 3 à 5 % du poids de la préparation.

7. Préparation cosmétique de bronzage selon l'une des revendications 1 à 6, caractérisée en ce que les liposomes représentent de 3 à 10 % du poids de la préparation.

8. Préparation cosmétique de bronzage selon l'une des revendications 1 à 7, caractérisée en ce que les composés filtres sont choisis dans le groupe comprenant les dérivés de l'acide cinamique, du benzylidène-camphre et de la benzophénone.

9. Préparation cosmétique de bronzage à base de liposomes dont la phase de dispersion contient des composés filtres et de la dihydroxyacétone (DHA), **caractérisée** en ce que :
   - la zone interne hydrophile des liposomes contient des principes hydratants, et de l'acétyltyrosine, ce dernier à raison de 0,1 à 1 % du poids de la préparation;
   - la paroi externe lipophile desdits liposomes contient de la DPHP.


## Patentansprüche

1. Kosmetisches Bräunungsmittel auf Basis von Liposomen, dessen Dispersionsphase filternde Verbindungen und Dihydroxyaceton (DHA) enthält, dadurch gekennzeichnet, daß der innere hydrophile Bereich der Liposomen Feuchtigkeitsstoffe und zumindest eine von Tyrosin abgeleitete Verbindung enthält, deren NH$_2$-Funktion durch eine chemische Gruppe blockiert ist.

2. Kosmetisches Bräunungsmittel nach Anspruch 1, dadurch gekennzeichnet, daß das Tyrosinderivat ausgewählt ist aus der Gruppe enthaltend Acetyltyrosin, Propionyltyrosin und Lipoyltyrosin.

3. Kosmetisches Bräunungsmittel nach Anspruch 2, dadurch gekennzeichnet, daß das Tyrosinderivat 0,1 bis 1 Gew.-%, vorzugsweise 0,5 Gew.-%, des Mittels darstellt.

4. Kosmetisches Bräunungsmittel nach einem der Ansprüche 1 bis 3, dadurch gekennzeichnet, daß die Feuchtigkeitsstoffe aus der Gruppe ausgewählt sind, die hygroskopische Substanzen , die die Austrocknung der Haut hemmen, enthält und zwar ausgewählt aus der Gruppe bestehend aus Polyolen, Harnstoff, Milchsäure, Pyrrolidoncarbonsäure und Kollagen.

5. Kosmetisches Bräunungsmittel nach einem der Ansprüche 1 bis 4, dadurch gekennzeichnet, daß die äußere lipophile Wand der Liposomen Dipalmitoylhydroxyprolin (DPHP) enthält.

6. Kosmetisches Bräunungsmittel nach einem der Ansprüche 1 bis 5, dadurch gekennzeichnet, daß das Dihydroxyaceton (DHA) auf 3 bis 5 Gew.-% des Mittels eingebracht ist.

7. Kosmetisches Bräunungsmittel nach einem der Ansprüche 1 bis 6, dadurch gekennzeichnet, daß die Liposomen 3 bis 10 Gew.-% des Mittels darstellen.

8. Kosmetisches Bräunungsmittel nach einem der Ansprüche 1 bis 7, dadurch gekennzeichnet, daß die filternden Verbindungen ausgewählt sind aus der Gruppe bestehend aus den Derivaten der Zimtsäure, des Benzylidencamphers und des Benzophenons.

9. Kosmetisches Bräunungsmittel auf Basis von Liposomen, dessen Dispersionsphase filternde Verbindungen und Dihydroxyaceton (DHA) enthält, dadurch gekennzeichnet, daß
   - der innere hydrophile Bereich der Liposomen Feuchtigkeitsstoffe und Acetyltyrosin enthält, wobei letzteres mit 0,1 bis 1 Gew.-% des Mittels enthalten ist; und
   - daß die äußere lipophile Wand der Liposomen DPHP enthält.


**Claims**

1. A sun tanning cosmetic preparation based on liposomes, and in which the dispersion phase contains screening compounds and dihydroxyacetone (DHA), characterized in that the hydrophilic internal region of the liposomes contains hydrating principles and at least one compound derived from tyrosine in which the $-NH_2$ function is blocked by a chemical radical.

2. The cosmetic tanning preparation as claimed in claim 1, wherein the tyrosine derivative is selected from the group comprising acetyltyrosine, proprionyltyrosine and lipoyltyrosine.

3. The cosmetic tanning preparation as claimed in claim 2, wherein the tyrosine derivative represents from 0.1 to 1 % by weight of the weight of the preparation, and preferably 0.5 %.

4. The cosmetic tanning preparation as claimed in one of claims 1 to 3, wherein the hydrating principles are selected from the group comprising hygroscopic substances retarding skin dehydration, selected from the group consisting of polyols, urea, lactic acid, pyrrolidonecarboxylic acid and collagen.

5. The cosmetic tanning preparation as claimed in one of claims 1 to 4, wherein the lipophilic external wall of the liposomes contains dipalmitoylhydroxyproline (DPHP).

6. The cosmetic tanning preparation as claimed in claims 1 to 5, wherein the dihydroxyacetone (DHA) is introduced in the proportion of 3 to 5 % of the weight of the preparation.

7. The cosmetic tanning preparation as claimed in one of claims 1 to 6, wherein the liposomes represent from 3 to 10 % of the weight of the preparation.

8. The cosmetic tanning preparation as claimed in one of claims 1 to 7, wherein the screening compounds are selected from the group comprising derivatives of cinnamid acid, of benzylidenecamphor and of benzophenone.

9. A cosmetic tanning preparation based on liposomes in which the dispersion phase contains screening compounds and dihydroxyacetone (DHA), wherein :
   - the hydrophilic internal region of the liposomes contains hydrating principles and acetyltyrosine, the latter in the proportion of 0.1 to 1 % of the weight of the preparation ;
   - the lipophilic external wall of said liposomes contains DPHP.